# EUROPEAN PATENT APPLICATION

(11) **EP 0 945 512 A2**
(43) Date of publication of application: **29.09.1999**
(21) Application number: 99301053.7
(22) Date of filing: 15.02.1999
(51) Int. Cl.: C12N 15/57, C12N 9/64, C12N 1/21, C12N 5/10, C07K 16/40, C12Q 1/37

(54) **Novel serine proteases**

(30) Priority: 13.02.1998 JP 3148798
(71) Applicant: SUNTORY LIMITED, Kita-ku, Osaka-shi, Osaka (JP)
(72) Inventor: Tsuruoka, Nobuo, Ibaraki-shi, Osaka (JP); Yamashiro, Kyoko, Takatsuki-shi, Osaka (JP); Mitsui, Shinichi, Kyoto-shi, Kyoto (JP); Yamaguchi, Nozomi, Karamaguchi-tori,Kita-ku, Kyoto-shi (JP)
(74) Representative: Stoner, Gerard Patrick

(57) **Abstract**

Polypeptides having the amino acid sequences listed as SEQ.NO.13 and 14 or those amino acid sequences modified by deletion, addition, substitution or a combination thereof of one or more amino acids in said amino acid sequence, and having serine protease activity; DNA coding therefor; and a method for producing the polypeptides using the DNA.

## Description

The present invention relates to novel serine proteases, DNA coding therefor and a method for producing the serine proteases, as well as to a screening method for physiologically active substances using the serine proteases or DNA coding therefor, and a drug therapy application using DNA fragments which suppress expression of the novel serine proteases.

### BACKGROUND

Serine proteases are known to be widely present in animals, plants and microorganisms, and in particularly they are known to be involved in many biological reactions in higher animals, including food digestion, blood coagulation and fibrinolysis, complement activation, hormone production, ovulation and fertilization, phagocytosis, cell proliferation, development and differentiation, aging, cancer metastasis, etc. (Neurath, H. Science, 224, 350-357, 1984). Recently it has been confirmed that serine proteases also act as physiologically important functional molecules in the central nervous system.

Serine proteases in the brain are believed to be not only involved in the promotion of neurite outgrowth of neurons, but are also to be involved in the process of synapse formation with target neurons (Liu, Y., Fields, R.D., Fitzgerald, S., Festoff, B.W., and Nelson, P.G., J. Neurobiol., 25, 325-, 1994). For example, it is known that both tissue (tPA) and urokinase (uPA) plasminogen activator (PA) are expressed in the brain, and experiments using cultured nerve cells have been reported which show that PA induces extension of neurite outgrowth (Pittman, R.N., Ivins, J.K. and Buettner, H.M., J. Neurosci., 9, 4269, 1989).

Experiments using cultured nerve cells have also shown that thrombin, which has also been confirmed to be expressed in the brain, contributes to the inhibitory effect of neurite extension in counteraction to PA (Dihanich, M., Kaser, M., Reinhard, E., Cunningham, D. and Monard, D., Neuron, 6, 575, 1991). Since thrombin receptor, that span the membrane seven times and coupled to G-Proteins, is expressed widely throughout the brain, and the inhibitory effect of thrombin on neurite extension also occurs by the amino-terminal peptides (tethered ligand) of the receptor with agonist activity, the inhibitory effect of thrombin on neurite outgrowth is believed to be a receptor-mediated specific response.

Elsewhere it has been reported that serine protease inhibitors (serpins) are also expressed in the brain, such as protease nexin I, for example, which acts as a powerful thrombin inhibitor preventing cellular death of motor neurons. It has also been reported that in some Alzheimer (AD) patients the amount of the α1-antichymotrypsin as a serpin is increased in the cerebrospinal fluid, while conversely the amount of protease nexin I which is also a serpin is reduced in the cerebrospinal fluid.

Such facts indicate a clear upset of the quantitative balance between serine proteases and serpins in the brains of AD patients. In particular, since the reduced amount of protease nexin I which is a powerful thrombin inhibitor would be expected to produce a state of relatively accelerated thrombin activity in the brain, this suggests that part of the pathological condition of AD patients is due to the inhibitory effect of thrombin on neurite outgrowth.

Based on these results, it may be concluded that the various neurodegenerative diseases involve imbalance in the serine proteases and serine protease inhibitors in the brain. Consequently, a therapy which controls such imbalance is necessary in these neurodegenerative disorders. However, it has been very difficult to explain all of the pathological conditions of neurodegenerative diseases in terms of only the presently known serine proteases among all of the serine proteases which are expressed and carry out important physiological functions in the brain. Also, while it is expected that numerous other serine proteases exist which are expressed in the brain and carry out important physiological functions, most of them have not yet been identified.

The present invention was accomplished in light of these circumstances, and its object is to provide DNA coding for novel serine proteases as well as the novel serine proteases themselves. It is a further object of the invention to provide a method for suppressing expression of the novel serine proteases by the antisense method using a fragment of the DNA, as well as a method of producing the serine proteases in large amounts and a method of screening for inhibitors of the serine proteases using the DNA, and to provide an assay system using specific antibodies for the serine proteases.

As a result of much diligent research, the present inventors have obtained full length cDNA coding for two different novel serine proteases, by direct isolation of cDNA coding for serine protease proteins expressed in the brain from human brain-derived mRNA by reverse transcription polymerase chain reaction (RT-PCR). We have also succeeded in expressing cDNA coding for the serine protease proteins in animal cells to achieve expression of the mature proteins, and have thus completed the present invention.

The present invention therefore provides a polypeptide having the amino acid sequence shown in SEQ. ID. NO:13 or an amino acid sequence modified by deletion, addition and/or substitution of one or more amino acids and having serine protease activity; a polypeptide having the amino acid sequence shown in SEQ. ID. NO:14 or an amino acid sequence modified by deletion, addition and/or substitution of one or more amino acids and having serine protease activity; and partial peptides thereof.

The present invention further provides a polypeptide encoded by naturally occurring DNA which hybridizes with DNA having the nucleotide sequence shown in SEQ. ID. NO:11 under stringent conditions, which has serine protease activity; a polypeptide encoded by naturally occurring DNA which hybridizes with DNA having the nucleotide sequence shown in SEQ. ID. NO:12 under stringent conditions, which has serine protease activity; and partial peptides thereof.

The present invention still further provides DNA coding for the aforementioned polypeptides, or fragments thereof. The DNA or fragments thereof are useful for producing the polypeptides or partial peptides thereof, and the DNA or fragments thereof are also useful for suppressing expression of serine proteases by the antisense method.

The present invention still further provides a vector, especially an expression vector, comprising the aforementioned DNA or its fragment, and a host transformed by the vector.

The present invention still further provides a method of producing the aforementioned polypeptides or partial peptides, which is characterized by culturing or breeding the aforementioned host and collecting the serine proteases or partial peptides thereof.

The present invention still further provides antibodies against the aforementioned polypeptides.

The present invention still further provides a screening method for physiologically active substances, using the aforementioned polypeptides or partial peptides thereof or the aforementioned DNA or fragments thereof.

### DETAILED DESCRIPTION

cDNA was synthesized from commercially available human brain-derived poly(A)+RNA, and subjected to PCR using a primer designed based on the serine protease motif. The resulting PCR product was sequenced, and cDNA fragments coding for novel serine proteases were obtained. The 5' end sequences and 3' end sequences were extended based on the obtained sequence data to elucidate the primary structure of the cDNA coding for the novel serine proteases.

It was then attempted to isolate the full length cDNA by the PCR method based on the elucidated structure. As a result, two types of cDNA were successfully isolated, and were designated as human SP66 type 1 and human SP66 type 2. Human SP66 type 1 and human SP66 type 2 were both shown to code for novel serine proteases. Examples 1, 2 and 3 are provided as concrete examples. The present inventors then used COS-1 cells to express the mature protein encoding the human SP66 type 1 and human SP66 type 2 cDNA, and confirmed that they were in fact functional proteins with enzyme activity. Example 4 is provided as a concrete example.

These results demonstrated that the isolated human SP66 type 1 and human SP66 type 2 coded for novel serine proteases which were not only novel in their primary structure but also were actually functional proteins with enzyme activity.

Since the novel serine proteases of the invention are functional proteins with enzyme activity, they carry out specific roles for physiological function in the brain through their enzyme activity. For the various neurodegenerative diseases where there is an imbalance between serine proteases and serine protease inhibitors in the brain, these serine proteases may be supplemented or expression of the serine proteases may be suppressed, in order to normalize the balance between serine proteases and serine protease inhibitors in the brain, as an effective method of medical treatment.

As particular treatments there may be mentioned intraventricular administration and forced expression by gene therapy as supplementation treatments, which would be based on increasing intracerebral levels for improvement of dementia. Also possible are inhibition therapies based on inhibiting agents created by using the cDNA or a fragment described in the present application to express a gene recombinant and screening for inhibitors. The cDNA of the present application may also be applied in gene therapy using antisense inhibition methods since it is characterized by being human-derived.

Furthermore, monitoring the balance between serine proteases and serine protease inhibitors in the brain is important for diagnosis of various neurodegenerative diseases and pathological analysis, which includes biochemical examinations, and this can be accomplished by quantifying the amount of the novel serine proteases of the invention in body fluids, and especially the cerebrospinal fluid. For example, by measuring the amount of the novel serine proteases of the invention in the cerebrospinal fluid it is possible to determine the amelioration or progression of dementia, and therefore the present invention also provides specific antibodies for the novel serine proteases of the invention and clinical examination kits for their measurement to determine therapeutic effects. In addition, since quantifying the amounts of the novel serine proteases of the invention in cerebrospinal fluid can serve to determine amelioration or progression of dementia, a major contribution is thus provided toward the development of drug treatments for diseases for which no method of treatment exists, such as Alzheimer's disease.

The present invention will now be explained in greater detail.

According to the invention there are disclosed the nucleotide sequences shown in SEQ. ID. NO:11 and SEQ. ID. NO:12 as nucleotide sequences of DNA coding for the novel serine proteases of the invention, but the DNA of the serine proteases of the invention is not necessarily restricted to them. Once the amino acid sequence of a natural serine protease has been determined, different nucleotide sequences coding for the same amino acid sequence can be designed and modified based on degeneration of the code. In such cases, a preferred embodiment is one which uses codons which are utilized with a high frequency by the intended host.

DNA coding for the natural-type serine proteases of the invention may be obtained in a manner of preparing cDNA such as described in the examples, but is not limited to this. In other words, DNA coding for the amino acid sequences of the natural serine proteases may be cloned as cDNA by a different strategy than the strategy specifically disclosed by the present invention, and it may also be cloned from the genome of cells which produce it.

The cloning may be accomplished by, for example, hybridization from a brain cDNA library. The DNA of the invention may also include DNA which codes for a protein or glycoprotein with serine protease activity and which hybridizes with the nucleotide sequence of SEQ. ID. NO:11 or SEQ. ID. NO:12 under stringent conditions. Such DNA is preferably naturally occurring cDNA or genomic DNA, and it may be human-derived DNA, for example. Common methods for hybridization are well known among those skilled in the art (such as Jikken Igaku Rinji Special Issue, Yodosha, Biotechnology Experiment Methods Series, Genetic Engineering Overview, Vol.5, No.11, 24-60, 1987; Current Protocols in Molecular Biology, John Wiley & Sons, Chap.6, 1995), and methods for measuring activity are also well known among those skilled in the art.

When cloning from a genome, the different primer nucleotides or probe nucleotides used in the examples may be used as probes for selection of genomic DNA fragments. Other probes may also be used which are designed based on the nucleotide sequences listed as SEQ. NO.11 and SEQ. NO.12. Common methods for cloning desired DNA from genomes are well known among those skilled in the art (Current Protocols In Molecular Biology, John Wiley & Sons, Chaps. 5 and 6, 1995).

DNA coding for the natural serine proteases of the invention can also be prepared by chemical synthesis. Chemical synthesis of DNA can be easily accomplished by a person skilled in the art using an automatic DNA synthesizer, such as the 396 DNA/RNA synthesizer by Applied Biosystems Co. Thus, a person skilled in the art can easily synthesize DNA having the nucleotide sequence shown in SEQ. ID. NO:11 or SEQ. ID. NO:12.

DNA which codes for naturally occurring serine proteases according to the invention with codons other than the original codons can be prepared by chemical synthesis as described above, and can be obtained by a conventional method such as site-directed mutagenesis using DNA or RNA with the nucleotide sequence shown in SEQ. ID. NO:11 or SEQ. ID. NO:12 as a template together with a mutagenic primer, according to a common method (see, for example, Current Protocols in Molecular Biology, John Wiley & Sons, Chap.8, 1995).

Once the amino acid sequence has been thus determined, it is possible to design and produce various different mutant serine proteases, such as polypeptides with one or more amino acid additions to the natural amino acid sequence which also maintain serine protease activity, polypeptides with one or more amino acid deletions from the natural amino acid sequence which also maintain serine protease activity, polypeptides with one or more substitutions of other amino acids for amino acids in the natural amino acid sequence which also maintain serine protease activity, and polypeptides with mutations involving combinations of amino acid addition mutations, amino acid deletion mutations and amino acid substitution mutations, which also maintain serine protease activity.

These may be prepared by the following method or by a peptide synthesis method which is known or by cleavage of the serine protease with an appropriate protease. Mutant serine proteases which maintain the serine protease activity of the invention or DNA coding therefor may also be prepared in the same manner.

There is no particular restriction on the number of amino acids involved in the addition, deletion or substitution of amino acids, and for additions it will be determined based on the purpose of the mutation, i.e. it will depend on the number of amino acids of a functional protein used as a hybrid protein with the serine protease of the invention, such as a protein for publicly known extraction and purification or stabilization (for example, maltose binding protein) or any of various physiologically active proteins, or on the number of amino acids of a signal peptide added to the serine protease. For example, 1 to 50, and preferably 1 to 10 additions may be made.

For deletions, the number of amino acids deleted may be for example 1 to 30, and preferably 1 to 20, as designed and determined for maintaining serine protease activity, or may be a number of amino acids in a region outside of the activity range of the serine protease. For substitutions, the number of substituted amino acids may be for example 1 to 10, and preferably 1 to 5, as designed and determined for maintaining serine protease activity.

It is preferred for the aforementioned mutated and modified serine proteases to have for example at least 70%, preferably at least 80% and more preferably at least 90% homology with the original amino acid sequences shown in SEQ. ID. NOS:13 and 14.

When DNA for a serine protease or mutant serine protease according to the invention has been obtained in this manner, it may be used to produce a gene recombinant serine protease or a gene recombinant mutant serine protease by any common method. Specifically, DNA coding for the serine protease or mutant serine protease of the invention may be inserted into an appropriate expression vector, the expression vector then introduced into appropriate host cells, the host cells then cultured and the desired serine protease or mutant serine protease collected from the obtained culture (cells or medium).

The serine protease or mutant serine protease of the invention may also be obtained in a biochemically modified form, for example by N-terminal acylation and C1-6 acylation, including formylation and acetylation, or in a deleted form. The expression system may be designed for secretion efficiency and expression volume by addition or improvement of the signal sequence or selection of the host. Means for addition and improvement of signal sequences include linkage of a gene coding for the signal peptide of another structural peptide upstream of the 5' end of the serine protease or mutant serine protease structural general of the invention, via a gene coding for a cleavable partial peptide. As a specific example there may be mentioned a method using the signal sequence of the trypsin gene mentioned in Example 4 and a gene coding for an enterokinase-recognizing sequence.

A eukaryotic or prokaryotic organism may be used as the host. Prokaryotic organisms which may be used include bacteria, especially *Escherichia coli* and *Bacillus* bacteria such as *B. subtilis.* Eukaryotic organisms which may be used include yeast, for example *Saccharomyces* yeast such as *S. cerevisiae* and other eukaryotic microorganisms, as well as insect cells such as *Spodoptera frugiperda, Trichoplusia ni, Bombyx mori,* and animal cells such as human cells, simian cells, mouse cells and the like, particularly COS cells, Vero cells, CHO cells, L cells, C127 cells, BALB/c 3T3 cells and Sp-2/0 cells.

The expression vector used may be a plasmid, phage, phagemid, virus (baculo (insect) or vaccinia (animal cells)), etc. The promoter in the expression vector is selected based on the host cells; examples of bacterial promoters include lac promoter and trp promoter, and examples of yeast promoters include adhI promoter and pqk promoter.

Insect promoters include baculovirus polyhedrin promoter, etc. and for animal cells the Simian Virus 40 early or late promoter, CMV promoter, HSV-TK promoter or SRα promoter may be mentioned. A preferred embodiment of the expression vector is one which further includes an enhancer, a splicing signal, a polyA addition signal, a selection marker (for example, a dihydrofolate reductase gene (methotrexate resistance) and a neo gene (G418 resistance)). When an enhancer is used, an SV40 enhancer, for example, may be inserted upstream or downstream of the gene.

Transformation of the host with the expression vector may be accomplished by a common method well known to those skilled in the art, and such methods are described for example in Current Protocols In Molecular Biology, John Wiley & Sons, 1995. The transformants may also be cultured according to common methods. purification of the serine protease or mutant serine protease from the cultured product may be accomplished according to common methods for isolation and purification of proteins, for example by ultrafiltration and different types of column chromatography, such as chromatography using Sepharose (Pharmacia Biotechnologies).

Because the serine protease or mutant serine protease of the invention obtained in this manner is a functional protein with enzyme activity, it can serve as a useful means for pathological analysis, and allows screening of physiologically active substances using the protein, by screening methods which are useful for investigational research on treatment drugs for various diseases.

As for one example of such screening methods there can be used to measure the physiological activity of test samples of peptides, proteins, non-peptide compounds, synthetic compounds and fermentation products, as well as of natural components which are obtained from culture supernatants and artificial components including various synthetic compounds.

Preferred embodiments of the screening method of the invention also include the aforementioned physiological activity measurement, as well as binding affinity assays, which employ DNA coding for the serine protease or partial peptide thereof or mutant serine protease or partial peptide thereof according to the invention, or a host transformed thereby or its cell membrane fraction (see Shinkiso Seikagaku Jikkenho 6, Assay Methods Using Biological Activity, ed. Nakajima, T., Nomoto, A., Matsuhashi, M., Miura, K., Muramatsu, M., Maruzen, KK., 1988).

That is, a screening method for physiologically active substances using a serine protease or its partial peptide according to the invention is carried out by screening a test sample using a serine protease or its partial peptide according to the invention, DNA coding therefor, or host cells or their cell membrane fraction containing the serine protease or its partial peptide. A specific method may be carried out by an enzyme activity assay (such as the modified method described in Example 4) or binding affinity assay using a substrate for the serine protease or its partial peptide according to the invention, for example a color-development substrate or other synthetic substrate, or a substrate labelled with a radio isotope.

When using host cells containing the serine proteases or their partial peptides, the cells may be immobilized by a publicly known method (with a reagent for example glutaraldehyde, formaldehyde, etc.). When using DNA coding for the proteases or their partial peptides, the accelerated or suppressed gene expression may be evaluated using a reporter gene, such as luciferase.

The aforementioned cell membrane fraction is the fraction containing most of the cell membrane, obtained by culturing host cells capable of expressing DNA coding for the serine protease or its partial peptide according to the invention under conditions suitable for expression, and then disrupting the host cells containing the serine protease or its partial peptide by a publicly known method.

The serine protease or its partial peptide or mutant serine protease or its partial peptide according to the invention, or DNA coding therefor, may also be provided in the form of a kit for use when carrying out the above-mentioned screening method.

In particular, since the serine proteases of the invention and DNA coding therefor are characterized by being human-derived, DNA coding for the serine protease or its partial peptide according to the invention may be used to produce a gene recombinant which may be directly administered to humans, for use as a supplementation treatment. DNA coding for a serine protease or its partial peptide according to the invention may also be administered directly to humans as a gene expression inhibiting treatment by the antisense method (Progress in Nucleic Acid Research and Molecular Biology, vol.44, p143-166, ed. by Cohn, W.E. and Moldave, K., "Cell Delivery and Mechanisms of Action of Antisense Oligonucleotides", Academic Press, Inc., 1993).

As partial peptides there may be mentioned serine protease fragments comprising regions unique to the serine proteases of the invention, such as peptide fragments present near the serine residues of the active sites and peptide fragments which can serve as recognition sites for antibodies specific to the serine proteases or mutated serine proteases of the invention. Such partial peptides can be prepared by the aforementioned methods for the serine proteases or mutated serine proteases of the invention or a publicly known peptide synthesis method, or by cleavage of the serine proteases or mutated serine proteases with appropriate proteases.

A partial peptide obtained in this manner may be bound to a carrier protein and then used to immunize a rabbit, mouse, rat, goat, chicken, guinea pig or other animal by a common method to obtain antiserum (polyclonal antibodies). Monoclonal antibodies may also be prepared by using a common method to immunize mice, rats or other animals and fusing the cells with myeloma cells to produce hybridomas (see Shinkiso Seikagaku Jikkenho 6, Assay Methods Using Biological Activity, ed. Nakajima, T., Nomoto, A., Matsuhashi, M., Miura, K., Muramatsu, M., Maruzen, KK., 1988).

In addition, DNA for the aforementioned serine proteases or mutant serine proteases may be used to produce gene recombinant serine proteases or gene recombinant mutant serine proteases which are then used to prepare polyclonal antibodies or monoclonal antibodies by similar methods.

Furthermore, the polyclonal antibodies or monoclonal antibodies obtained in this manner can be used for assay of the serine proteases of the invention in human body fluids, to allow pathological analyses including diagnoses and biochemical examinations for various neurodegenerative diseases.

### EXAMPLES

The present invention will now be explained in fuller detail by way of examples.

### Example 1. RT-PCR using serine protease-conserved region

Human brain poly(A)+RNA (Code No. CL6516-1) purchased from Clontech Co. was used for RT-PCR with the serine protease-conserved region. That is, 2 µl (1 µg) of oligo dT primer was added to 5 µl (about 6 µg) of mRNA, and after heating at 70°C for 10 minutes the mixture was cooled on ice. To this heat-denatured mRNA there were added 4 µl of 5 x first-strand buffer solution (250 mM Tris/HCl, pH 8.3, 375 mM KCl, 15 mM MgCl₂), 1 µl of 10 mM dNTP, 2 µl of 0.1 M DTT, distilled water treated with diethylpyrocarbonate (DEPC) and 5 µl (1000 U) of SuperScript II RT, and reaction was conducted at 37°C for one hour.

The first strand cDNA obtained in this manner was used as a template for PCR with the serine protease-conserved region. The primers used were the oligomer 5' GTG CTC ACN GCN GCB CAY TG 3' shown in SEQ. ID. NO: 1 synthesized based on the amino acid-conserved region (N-Val-Leu-Thr-Ala-Ala-His-Cys) near an active residue (His) and the oligomer 5' AGC GGN CCN CCD SWR TCV CC 3' shown in SEQ. ID. NO: 2 synthesized based on the amino acid-conserved region (N-Gly-Asp-Ser-Gly-Gly-Pro-Leu) near an active residue (Ser). Taq DNA polymerase (Amersham Co.) was used for PCR, after which the PCR reaction solution was subcloned in a pCR II vector (Invitrogen Co.). Sequencing of the resulting PCR product by a common method confirmed a cDNA fragment coding for a novel serine protease.

### Example 2. Obtaining 5' end and 3' end sequences

### Step 1. cDNA synthesis

A 5 µl (approximately 5 µg) portion of the same human brain poly(A)+RNA in Example 1 was used to synthesize template cDNA. Specifically, after adding 2 µl (1 µg) of oligo dT adapter primer and heating at 70°C for 10 minutes, the mixture was cooled on ice. To this heat-denatured mRNA there were added 4 µl of 5 x first-strand buffer solution (250 mM Tris/HCl, pH 8.3, 375 mM KCl, 15 mM MgCl₂), 1 µl of 10 mM dNTP, 2 µl of 0.1 M DTT, distilled water treated with DEPC and 5 µl (1000 U) of SuperScript II RT, and reaction was conducted at 37°C for one hour.

Next, 91 µl of distilled water treated with DEPC, 30 µl of 5 x second-strand buffer solution (100 mM Tris-HCl, pH 6.9, 450 mM KCl, 23 mM MgCl₂, 0.75 mM β-NAD+, 50 mM (NH₄)₂SO₄), 3 µl of 10 mM dNTP, 1 µl (10 U) of *E. coli* DNA ligase, 4 µl (40 U) of *E. coli* DNA polymerase and 1 µl (2 U) of *E. coli* RNase H were added to the reaction solution, and after reaction at 16°C for 2 hours, 2 µl (10 U) of T4 DNA polymerase was added and reaction was continued at 16°C for 5 minutes. A 10 µl portion of 0.5 M EDTA was added to and mixed with the solution, 150 µl of phenol:chloroform:isoamyl alcohol (25:24:1) was added, and after stirring the mixture it was centrifuged at 15,000 rpm for 5 minutes and the supernatant was recovered. To this supernatant there were added 10 µl of 5 M KOAc and 400 µl of ethanol, and the mixture was stirred and centrifuged at 15,000 rpm for 10 minutes. The precipitate was washed with 500 µl of 70% ethanol, lightly air dried and dissolved in 25 µl of distilled water treated with DEPC.

### Step 2. Addition of adaptor for obtaining 5' end sequence

To 25 µl of the double-stranded cDNA obtained in step 1 there were added 10 µl of 5 x T4 DNA ligase buffer solution (250 mM Tris/HCl, pH 7.6, 50 mM MgCl₂, 5 mM ATP, 5 mM DTT, 25% (w/v) PEG 8000), 10 µl (10 µg) of a 5' RACE adapter solution and 5 µl (5 U) of T4 DNA ligase, and after reaction at 16°C for 16 hours, 50 µl of phenol:chloroform:isoamyl alcohol (25:24:1) was added, the mixture was stirred and then centrifuged at 15,000 rpm for 5 minutes and the supernatant was recovered. To this supernatant there were added 5 µl of 5 M KOAc and 125 µl of ethanol, and after stirring the mixture and cooling it at -80°C for 20 minutes, it was centrifuged at 15,000 rpm for 10 minutes. The precipitate was washed with 200 µl of 70% ethanol, lightly air dried and then dissolved in 40 µl of distilled water treated with DEPC.

### Step 3. Obtaining 5' end sequence and 3' end sequence

The antisense primer 5' ACCTTCTTAAGCGCCGGCGTC 3' listed as SEQ. NO.3 was synthesized based on the oligo dT primer sequence used in step 1 to obtain the 3' end sequence. Also, the primer 5' TCAGCCTGGCAGATCATTGC 3' shown in SEQ. ID. NO: 4 and the primer 5' CAGAAGTGCACCGTCTCAGG 3' shown in SEQ. ID. NO: 5 which were specific to the sequences obtained in Example 1 were synthesized as sense primers. The sense primer 5' ACTCACTATAGGGCTCGAGCGGC 3' shown in SEQ. ID. NO: 6 was also synthesized based on the adapter sequence added in step 2 to obtain the 5' end sequence.

The primer 5' ACAGGTATTTCTTGCTCTGGG 3' shown in SEQ. NO.7 and the primer 5' TTCTGTAGGCTGTGGTCTCCC 3' which were specific to the sequences obtained in Example 1 were also synthesized as antisense primers. The cDNA obtained in step 1 was used as a template for PCR using the set of primers shown in SEQ. NO. 3 and 4 and the set of primers shown in SEQ. ID. NO: 6 and 7. The resulting PCR product was diluted 100-fold with TE solution and 0.5 µl of the dilution was used as a template for PCR using the set of primers listed as SEQ. NO. 3 and 5 and the set of primers listed as SEQ. NO. 6 and 8. The resulting PCR product was subcloned in a PCR II vector (Invitrogen Co.). The base sequence was determined according to a common method.

### Example 3. Obtaining full length cDNA for novel serine proteases

The following primers were synthesized based on the sequences obtained in Example 2.

That is, the primer 5' GGATTCTGGAAGACCTCACCACC 3' shown in SEQ. ID. NO: 9 and the primer 5'
CCATGGACGTCCCAGAGAGTTGTGAGTTTATTAAG 3' shown in SEQ. ID. NO: 10 were used as a set of primers for PCR with the single-stranded cDNA obtained in Example 1 as the template. The resulting product was subcloned in a pCR II vector (Invitrogen Co.). The base sequence was determined according to a common method.

As a result, the two types of cDNA shown in SEQ. ID. NO:11 and 12 were obtained. The cDNA with a full length of 905 base pairs was designated as SP66 type 1, and the cDNA with a full length of 942 base pairs was designated as SP66 type 2. SP66 type 1 consists of a 54 bp 5' untranslated region, a 783 bp translated region and a 68 bp 3' untranslated region, and the translated region codes for a novel serine protease (260 amino acids). SP66 type 2 has a 918 bp translated region, and the translated region codes for a novel serine protease (305 amino acids). Also, SP66 type 1 and SP66 type 2 were shown to code for a common mature protein (mature serine protease) consisting of 228 amino acids.

### Example 4. Assay of novel serine protease mature protein coded for by SP66 type 1 and SP66 type 2

### (1) Construction of expression plasmid

A pUC18/SP66 type 1 DNA fragment and a pdKCR vector DNA fragment were ligated by a common method and used to transform *E. coli* JM109, and the resulting colonies were analyzed by PCR to obtain the target serine protease SP66 expression plasmid pdKCR/SP66. Next, cDNA coding for the signal sequence of trypsin II following the initiating methionine and for an enterokinase-recognizing sequence was amplified, and primers were designed with EcoRI added upstream of the 5' end and a BspMI restriction endonuclease-recognition site downstream of the 3' end. These primers were used for PCR with the pCR/Trypsin II plasmid as the template, and after digesting the product with restriction endonucleases (EcoRI and BspMI), an approximately 75 bp DNA fragment was isolated and purified.

Similarly, a primer designed with a BspMI restriction endonuclease-recognition site added upstream of the DNA coding for the SP66 mature protein was used as a primer for PCR with pdKCR/SP66 as the template, and after digesting the product with restriction endonucleases (BspMI and Bpu 1102I), a DNA fragment was isolated and purified. Next, the obtained cDNA fragment coding for the signal sequence of trypsin II and for an enterokinase-recognizing sequence and a cDNA fragment coding for the SP66 mature protein were ligated with the pdKCR/SP66 vector which had been predigested with restriction endonucleases (BspMI and Bpu 1102I) and used to transform *E. coli* JM109. Colonies containing the target chimeric DNA among the transformed colonies were verified by PCR to obtain an expression plasmid (pdKCR/Trp66).

### (2) Expression in COS-1 cells

The expression plasmid constructed in Example 4 (1) was used for transfection in COS-1 cells using lipofectin (Life Technologies). That is, 5 x 10⁵ COS-1 cells were implanted in 10-cm diameter culturing dishes (Corning, 430167) with Dulbecco's minimum essential medium (DMEM, Nissui Seiyaku) containing 10% fetal bovine serum. On the following day, the cells were rinsed with 5 ml of Opti-MEM medium (Life Technologies), after which 5 ml of Opti-MEM medium was freshly added for culturing at 37°C for 2 hours.

After the culturing, a mixture of 1 µg of the aforementioned plasmid and 5 µg of lipofectin was added to each dish, and the culturing was continued at 37°C for 5 hours. After this culturing, 5 ml of Opti-MEM medium was added, for a total of 10 ml, and culturing was continued at 37°C for 72 hours. After this culturing, the culturing supernatant obtained by centrifugation was collected and used as the enzyme activity assay sample. As a control, a culture supernatant was prepared from transfection of the expression plasmid pdKCR alone into COS-1 cells.

### (3) Measurement of enzyme activity

The enzyme activity in the culture supernatant obtained in Example 4 (2) was measured. That is, 5 µl of enterokinase (10 mg/ml, Biozyme Laboratories) was mixed with 45 µl of the COS-1 cell culture supernatant, and reacted at room temperature for 15 minutes. Next, 50 µl of a 0.2 M substrate solution containing the synthetic substrate Boc-Phe-Ser-Arg-MCA (Peptide Laboratories) dissolved in DMSO and diluted with 0.1 M Tris/HCl at pH 8.0 was added, and this was reacted at 37°C for one hour. After the reaction, the fluorescence was measured at an excitation wavelength of 485 nm and a fluorescent wavelength of 535 nm. As a result, enzyme activity was found only when the Trp66-expressing COS-1 cell culture supernatant was digested with enterokinase.

This result demonstrated that the serine protease encoded by SP66 is a functional protein with enzyme activity.

The novel serine proteases of the present invention carry out a specific role in the physiological functions of the brain through their enzyme function, and thus for the various neurodegenerative diseases where there is an imbalance between serine proteases and serine protease inhibitors in the brain, these serine proteases may be supplemented or expression of the serine proteases may be suppressed, in order to normalize the balance between the serine proteases and serine protease inhibitors in the brain, as an effective method of treatment.

As particular treatments there may be mentioned intraventricular administration and forced expression by gene therapy as supplementation treatments, which would be based on increasing intracerebral levels for improvement of dementia. Also possible are inhibition therapies based on inhibiting agents created by using the cDNA or a fragment described in the present application to express a gene recombinant and screening for inhibitors. The cDNA of the present application may also be applied in gene therapy using antisense inhibition methods since it is characterized by being human-derived.

Furthermore, monitoring the balance between serine proteases and serine protease inhibitors in the brain is important for diagnosis of various neurodegenerative diseases and pathological analysis, which includes biochemical examinations, and this can be accomplished by quantifying the amount of the novel serine proteases of the invention in body fluids, and especially the cerebrospinal fluid. For example, by measuring the amount of the novel serine proteases of the invention in the cerebrospinal fluid it is possible to determine the amelioration or progression of dementia, and according to the invention there are also provided specific antibodies for the novel serine proteases of the invention and clinical examination kits for their measurement to determine therapeutic effects. In addition, since quantifying the amounts of the novel serine proteases of the invention in cerebrospinal fluid can serve to determine amelioration or progression of dementia, a major contribution is provided toward the development of drug treatments for diseases for which no method of treatment exist, such as Alzheimer's disease.

## Claims

1. A polypeptide having the amino acid sequence shown in SEQ. ID. NO:13, or an amino acid sequence modified by deletion, addition, substitution or a combination thereof of one or more amino acids in said amino acid sequence and having serine protease activity; or a partial peptide thereof.

2. A polypeptide having the amino acid sequence shown in SEQ. ID. NO:14, or an amino acid sequence modified by deletion, addition, substitution or a combination thereof of one or more amino acids in said amino acid sequence and having serine protease activity; or a partial peptide thereof.

3. A polypeptide encoded by naturally occurring DNA which hybridizes with DNA having the nucleotide sequence shown in SEQ. ID. NO:11 under stringent conditions, which has serine protease activity.

4. A polypeptide encoded by naturally occurring DNA which hybridizes with DNA having the nucleotide sequence shown in SEQ. ID. NO:12 under stringent conditions, which has serine protease activity.

5. A polypeptide according to any one of claims 1 to 4, having an amino acid sequence with at least 70% homology with respect to the amino acid sequence shown in SEQ. ID. NO:13 or SEQ. ID. NO:14.

6. A polypeptide according to claim 5, having an amino acid sequence with at least 80% homology with respect to the amino acid sequence shown in SEQ. ID. NO:13 or SEQ. ID. NO:14.

7. A polypeptide according to claim 6, having an amino acid sequence with at least 90% homology with respect to the amino acid sequence shown in SEQ. ID. NO:13 or SEQ. ID. NO:14.

8. DNA coding for a polypeptide or partial peptide according to any one of claims 1 to 7, or a fragment thereof.

9. DNA according to claim 8 which suppresses expression of serine protease by the antisense method, or a fragment thereof.

10. A vector comprising DNA or a fragment according to claim 8 or 9.

11. A host transformed by an expression vector according to claim 10.

12. A method for producing a polypeptide or partial peptide according to any one of claims 1 to 7, characterized by culturing or breeding a host according to claim 11 and collecting the polypeptide or partial peptide thereof.

13. An antibody which recognize a polypeptide or partial peptide according to any one of claims 1 to 7.

14. A screening method for physiologically active substances, using a polypeptide or partial peptide according to any one of claims 1 to 7, or DNA or its fragment according to claim 8 or 9.


### Annex to the description

SEQUENCE LISTING

<110> SUNTORY LIMITED

<120> Novel serine proteases

<130> GPS/FP5755491

<140> EP 99301053.7
<141> 1999-02-15

<150> JP 10-031487
<151> 1998-02-13

<160> 14

<210> 1
<211> 20
<212> DNA
<213> Artificial Sequence

<220>
<223> Description of Artificial Sequence: Primer

<220>
<221> misc_feature
<222> (9, 12)
<223> n is a or g or c or t

<400> 1
gtgctcacng cngcbcaytg                                    20

<210>  2
<211>  20
<212>  DNA
<213>  Artificial Sequence

<220>
<223> Description of Artificial Sequence: Primer

<220>
<221> misc_feature
<222> (6, 9)
<223> n is a or g or c or t

<400>  2
agcggnccnc cdswrtcvcc                                          20

<210>     3
<211>     21
<212>     DNA
<213>     Artificial Sequence

<220>
<223> Description of Artificial Sequence: Primer

<400>     3
accttcttaa gcgccggcgt c                                        21

<210>     4
<211>     20
<212>     DNA
<213>     Artificial Sequence

<220>
<223> Description of Artificial Sequence: Primer

<400>     4
tcagcctggc agatcattgc                                          20

<210>	5
<211>	20
<212>	DNA
<213>	Artificial Sequence

<220>
<223> Description of Artificial Sequence: Primer

<400>	5
cagaagtgca ccgtctcagg	20

<210>	6
<211>	23
<212>	DNA
<213>	Artificial Sequence

<220>
<223> Description of Artificial Sequence: Primer

<400>	6
actcactata gggctcgagc ggc	23

<210>	7
<211>	21
<212>	DNA
<213>	Artificial Sequence

<220>
<223> Description of Artificial Sequence: Primer

<400>	7
acaggtattt cttgctctgg g	21

<210> 8
<211> 21
<212> DNA
<213> Artificial Sequence

<220>
<223> Description of Artificial Sequence: Primer

<400> 8
ttctgtaggc tgtggtctcc c 21

<210> 9
<211> 23
<212> DNA
<213> Artificial Sequence

<220>
<223> Description of Artificial Sequence: Primer

<400> 9
ggattctgga agacctcacc acc 23

<210> 10
<211> 35
<212> DNA
<213> Artificial Sequence

<220>
<223> Description of Artificial Sequence: Primer

<400> 10
ccatggacgt cccagagagt tgtgagttta ttaag 35

<210>     11

<211>     905

<212>     DNA

<213> Homo sapiens

<223>     Nucleotide sequence of cDNA coding for a serine
          protease

<400>     11

cactgggtcc gaatcagtag gtgaccccgc ccctggattc tggaagacct cacc atg  57
                                                            Met
                                                            1
gga cgc ccc cga cct cgt gcg gcc aag acg tgg atg ttc ctg ctc ttg  105
Gly Arg Pro Arg Pro Arg Ala Ala Lys Thr Trp Met Phe Leu Leu Leu
            5                   10                  15
ctg ggg gga gcc tgg gca gga cac tcc agg gca cag gag gac aag gtg  153
Leu Gly Gly Ala Trp Ala Gly His Ser Arg Ala Gln Glu Asp Lys Val
        20                  25                  30
ctg ggg ggt cat gag tgc caa ccc cat tcg cag cct tgg cag gcg gcc  201
Leu Gly Gly His Glu Cys Gln Pro His Ser Gln Pro Trp Gln Ala Ala
    35                  40                  45
ttg ttc cag ggc cag caa cta ctc tgt ggc ggt gtc ctt gta ggt ggc  249
Leu Phe Gln Gly Gln Gln Leu Leu Cys Gly Gly Val Leu Val Gly Gly
 50                  55                  60                  65
aac tgg gtc ctt aca gct gcc cac tgt aaa aaa ccg aaa tac aca gta  297
Asn Trp Val Leu Thr Ala Ala His Cys Lys Lys Pro Lys Tyr Thr Val
                70                  75                  80
cgc ctg gga gac cac agc cta cag aat aaa gat ggc cca gag caa gaa  345
Arg Leu Gly Asp His Ser Leu Gln Asn Lys Asp Gly Pro Glu Gln Glu
            85                  90                  95
ata cct gtg gtt cag tcc atc cca cac ccc tgc tac aac agc agc gat  393
Ile Pro Val Val Gln Ser Ile Pro His Pro Cys Tyr Asn Ser Ser Asp
        100                 105                 110
gtg gag gac cac aac cat gat ctg atg ctt ctt caa ctg cgt gac cag  441
Val Glu Asp His Asn His Asp Leu Met Leu Leu Gln Leu Arg Asp Gln
    115                 120                 125

gca tcc ctg ggg tcc aaa gtg aag ccc atc agc ctg gca gat cat tgc 489
Ala Ser Leu Gly Ser Lys Val Lys Pro Ile Ser Leu Ala Asp His Cys
130                 135                 140                 145
acc cag cct ggc cag aag tgc acc gtc tca ggc tgg ggc act gtc acc 537
Thr Gln Pro Gly Gln Lys Cys Thr Val Ser Gly Trp Gly Thr Val Thr
                150                 155                 160
agt ccc cga gag aat ttt cct gac act ctc aac tgt gca gaa gta aaa 585
Ser Pro Arg Glu Asn Phe Pro Asp Thr Leu Asn Cys Ala Glu Val Lys
            165                 170                 175
atc ttt ccc cag aag aag tgt gag gat gct tac ccg ggg cag atc aca 633
Ile Phe Pro Gln Lys Lys Cys Glu Asp Ala Tyr Pro Gly Gln Ile Thr
        180                 185                 190
gat ggc atg gtc tgt gca ggc agc agc aaa ggg gct gac acg tgc cag 681
Asp Gly Met Val Cys Ala Gly Ser Ser Lys Gly Ala Asp Thr Cys Gln
    195                 200                 205
ggc gat tct gga ggc ccc ctg gtg tgt gat ggt gca ctc cag ggc atc 729
Gly Asp Ser Gly Gly Pro Leu Val Cys Asp Gly Ala Leu Gln Gly Ile
210                 215                 220                 225
aca tcc tgg ggc tca gac ccc tgt ggg agg tcc gac aaa cct ggc gtc 777
Thr Ser Trp Gly Ser Asp Pro Cys Gly Arg Ser Asp Lys Pro Gly Val
                230                 235                 240
tat acc aac atc tgc cgc tac ctg gac tgg atc aag aag atc ata ggc 825
Tyr Thr Asn Ile Cys Arg Tyr Leu Asp Trp Ile Lys Lys Ile Ile Gly
            245                 250                 255
agc aag ggc tgattctagg ataagcacta gatctccctt aataaactca caactct 881
Ser Lys Gly
        260
ctggaaaaaa aaaaaaaaaa aaaa 905

<210> 12
<211> 942
<212> DNA
<213> Homo sapiens

<223> Nucleotide sequence of cDNA coding for a serine
protease

<400>    12

atg gga cgc ccc cga cct cgt gcg gcc aag acg tgg atg ttc ctg ctc    48
Met Gly Arg Pro Arg Pro Arg Ala Ala Lys Thr Trp Met Phe Leu Leu
1               5                   10                  15

ttg ctg ggg gga gcc tgg gca cgt gtg gaa gcc tgg acc tcc tca cta    96
Leu Leu Gly Gly Ala Trp Ala Ala Cys Gly Ser Leu Asp Leu Leu Thr
            20                  25                  30

agg gac act cca ggg cac agg agg aca agg tgc tgg ggt tgt atg cgg   144
Lys Lys Tyr Ala Glu Asn Leu Pro Cys Val His Leu Asn Pro Gln Trp
        35                  40                  45

aga act tgc cgt gtg tcc att tga acc cac agt ggc ctt ccc agc cct   192
Pro Ser Gln Pro Ser His Cys Pro Arg Gly Trp Arg Ser Asn Pro Leu
    50                  55                  60

cgc act gcc cca gag ggt ggc gat cca acc ctc tcc ctc ctg ctg cag   240
Pro Pro Ala Ala Gly His Ser Arg Ala Gln Glu Asp Lys Val Leu Gly
65                  70                  75                  80

ggt cat gag tgc caa ccc cat tcg cag cct tgg cag gcg gcc ttg ttc   288
Gly His Glu Cys Gln Pro His Ser Gln Pro Trp Gln Ala Ala Leu Phe
                85                  90                  95

cag ggc cag caa cta ctc tgt ggc ggt gtc ctt gta ggt ggc aac tgg   336
Gln Gly Gln Gln Leu Leu Cys Gly Gly Val Leu Val Gly Gly Asn Trp
            100                 105                 110

gtc ctt aca gct gcc cac tgt aaa aaa ccg aaa tac aca gta cgc ctg   384
Val Leu Thr Ala Ala His Cys Lys Lys Pro Lys Tyr Thr Val Arg Leu
        115                 120                 125

gga gac cac agc cta cag aat aaa gat ggc cca gag caa gaa ata cct   432
Gly Asp His Ser Leu Gln Asn Lys Asp Gly Pro Glu Gln Glu Ile Pro
    130                 135                 140

gtg gtt cag tcc atc cca cac ccc tgc tac aac agc agc gat gtg gag   480
Val Val Gln Ser Ile Pro His Pro Cys Tyr Asn Ser Ser Asp Val Glu
145                 150                 155                 160

gac cac aac cat gat ctg atg ctt ctt caa ctg cgt gac cag gca tcc   528
Asp His Asn His Asp Leu Met Leu Leu Gln Leu Arg Asp Gln Ala Ser
                165                 170                 175

ctg ggg tcc aaa gtg aag ccc atc agc ctg gca gat cat tgc acc cag   576
Leu Gly Ser Lys Val Lys Pro Ile Ser Leu Ala Asp His Cys Thr Gln
            180                 185                 190

cct ggc cag aag tgc acc gtc tca ggc tgg ggc act gtc acc agt ccc 624
Pro Gly Gln Lys Cys Thr Val Ser Gly Trp Gly Thr Val Thr Ser Pro
        195                 200                 205
cga gag aat ttt cct gac act ctc aac tgt gca gaa gta aaa atc ttt 672
Arg Glu Asn Phe Pro Asp Thr Leu Asn Cys Ala Glu Val Lys Ile Phe
    210                 215                 220
ccc cag aag aag tgt gag gat gct tac ccg ggg cag atc aca gat ggc 720
Pro Gln Lys Lys Cys Glu Asp Ala Tyr Pro Gly Gln Ile Thr Asp Gly
225                 230                 235                 240
atg gtc tgt gca ggc agc agc aaa ggg gct gac acg tgc cag ggc gat 768
Met Val Cys Ala Gly Ser Ser Lys Gly Ala Asp Thr Cys Gln Gly Asp
                245                 250                 255
tct gga ggc ccc ctg gtg tgt gat ggt gca ctc cag ggc atc aca tcc 816
Ser Gly Gly Pro Leu Val Cys Asp Gly Ala Leu Gln Gly Ile Thr Ser
            260                 265                 270
tgg ggc tca gac ccc tgt ggg agg tcc gac aaa cct ggc gtc tat acc 864
Trp Gly Ser Asp Pro Cys Gly Arg Ser Asp Lys Pro Gly Val Tyr Thr
        275                 280                 285
aac atc tgc cgc tac ctg gac tgg atc aag aag atc ata ggc agc aag 912
Asn Ile Cys Arg Tyr Leu Asp Trp Ile Lys Lys Ile Ile Gly Ser Lys
    290                 295                 300
ggc tgattctagg ataagcacta gatctcc 942
Gly
305

<210> 13
<211> 260
<212> PRT
<213> Homo sapiens

<223> Amino acid sequence of serine protease

<400> 13

Met Gly Arg Pro Arg Pro Arg Ala Ala Lys Thr Trp Met Phe Leu Leu
1               5                   10                  15

Leu Leu Gly Gly Ala Trp Ala Gly His Ser Arg Ala Gln Glu Asp Lys
            20                  25                  30

Val Leu Gly Gly His Glu Cys Gln Pro His Ser Gln Pro Trp Gln Ala
        35                  40                  45

Ala Leu Phe Gln Gly Gln Gln Leu Leu Cys Gly Gly Val Leu Val Gly
    50                  55                  60

Gly Asn Trp Val Leu Thr Ala Ala His Cys Lys Lys Pro Lys Tyr Thr
65                  70                  75                  80

Val Arg Leu Gly Asp His Ser Leu Gln Asn Lys Asp Gly Pro Glu Gln
                85                  90                  95

Glu Ile Pro Val Val Gln Ser Ile Pro His Pro Cys Tyr Asn Ser Ser
            100                 105                 110

Asp Val Glu Asp His Asn His Asp Leu Met Leu Leu Gln Leu Arg Asp
        115                 120                 125

Gln Ala Ser Leu Gly Ser Lys Val Lys Pro Ile Ser Leu Ala Asp His
    130                 135                 140

Cys Thr Gln Pro Gly Gln Lys Cys Thr Val Ser Gly Trp Gly Thr Val
145                 150                 155                 160

Thr Ser Pro Arg Glu Asn Phe Pro Asp Thr Leu Asn Cys Ala Glu Val
                165                 170                 175

Lys Ile Phe Pro Gln Lys Lys Cys Glu Asp Ala Tyr Pro Gly Gln Ile
            180                 185                 190

Thr Asp Gly Met Val Cys Ala Gly Ser Ser Lys Gly Ala Asp Thr Cys
        195                 200                 205

Gln Gly Asp Ser Gly Gly Pro Leu Val Cys Asp Gly Ala Leu Gln Gly
    210                 215                 220

Ile Thr Ser Trp Gly Ser Asp Pro Cys Gly Arg Ser Asp Lys Pro Gly
225                 230                 235                 240

Val Tyr Thr Asn Ile Cys Arg Tyr Leu Asp Trp Ile Lys Lys Ile Ile
                245                 250                 255

Gly Ser Lys Gly
            260

<210>    14
<211>    305
<212>    PRT
<213> Homo sapiens

<223>    Amino acid sequence of serine protease

<400>    14

Met Gly Arg Pro Arg Pro Arg Ala Ala Lys Thr Trp Met Phe Leu Leu
  1               5                  10                  15
Leu Leu Gly Gly Ala Trp Ala Ala Cys Gly Ser Leu Asp Leu Leu Thr
             20                  25                  30
Lys Lys Tyr Ala Glu Asn Leu Pro Cys Val His Leu Asn Pro Gln Trp
        35                  40                  45
Pro Ser Gln Pro Ser His Cys Pro Arg Gly Trp Arg Ser Asn Pro Leu
    50                  55                  60
Pro Pro Ala Ala Gly His Ser Arg Ala Gln Glu Asp Lys Val Leu Gly
 65                  70                  75                  80
Gly His Glu Cys Gln Pro His Ser Gln Pro Trp Gln Ala Ala Leu Phe
                 85                  90                  95
Gln Gly Gln Gln Leu Leu Cys Gly Gly Val Leu Val Gly Gly Asn Trp
            100                 105                 110
Val Leu Thr Ala Ala His Cys Lys Lys Pro Lys Tyr Thr Val Arg Leu
        115                 120                 125
Gly Asp His Ser Leu Gln Asn Lys Asp Gly Pro Glu Gln Glu Ile Pro
    130                 135                 140
Val Val Gln Ser Ile Pro His Pro Cys Tyr Asn Ser Ser Asp Val Glu
145                 150                 155                 160

Asp His Asn His Asp Leu Met Leu Leu Gln Leu Arg Asp Gln Ala Ser
                165                 170                 175
Leu Gly Ser Lys Val Lys Pro Ile Ser Leu Ala Asp His Cys Thr Gln
            180                 185                 190
Pro Gly Gln Lys Cys Thr Val Ser Gly Trp Gly Thr Val Thr Ser Pro
        195                 200                 205
Arg Glu Asn Phe Pro Asp Thr Leu Asn Cys Ala Glu Val Lys Ile Phe
    210                 215                 220
Pro Gln Lys Lys Cys Glu Asp Ala Tyr Pro Gly Gln Ile Thr Asp Gly
225                 230                 235                 240
Met Val Cys Ala Gly Ser Ser Lys Gly Ala Asp Thr Cys Gln Gly Asp
                245                 250                 255
Ser Gly Gly Pro Leu Val Cys Asp Gly Ala Leu Gln Gly Ile Thr Ser
            260                 265                 270
Trp Gly Ser Asp Pro Cys Gly Arg Ser Asp Lys Pro Gly Val Tyr Thr
        275                 280                 285
Asn Ile Cys Arg Tyr Leu Asp Trp Ile Lys Lys Ile Ile Gly Ser Lys
    290                 295                 300
Gly
305